# EUROPEAN PATENT SPECIFICATION

(11) **EP 3 228 614 B1**
(45) Date of publication and mention of the grant of the patent: **11.11.2020**
(21) Application number: 15865915.1
(22) Date of filing: 13.11.2015
(51) Int. Cl.: C07C 311/46, C07C 303/40, G03F 7/027

(54) **BENZYLSULFAMIDE SUBSTITUTED ACRYLAMIDE DERIVATIVE**
BENZYLSULFAMIDSUBSTITUIERTES ACRYLAMID-DERIVAT
DÉRIVÉ D'ACRYLAMIDE SUBSTITUÉ PAR BENZYLSULFAMIDE

(30) Priority: 03.12.2014 CN 201410728302
(43) Date of publication of application: 11.10.2017
(73) Proprietor: Qingdao Lanfan Advanced Materials Co. Ltd., Qingdao, Shandong 266000 (CN)
(72) Inventor: LIU, Yang, Qingdao Shandong 266000 (CN)
(74) Representative: Casalonga
(86) International application number: PCT/CN2015/094556
(87) International publication number: WO 2016/086762

(56) References cited:
- EP-A1- 2 263 874
- EP-A1- 3 236 315
- CN-A- 102 458 855
- CN-A- 102 504 137
- CN-A- 102 504 137
- CN-A- 104 402 778
- CN-A- 104 503 204
- JP-A- S60 185 754

## Description

### FIELD OF INVENTION

The invention belongs to the field of chemical synthesis, and particularly to a toluenesulfonamide-substituted acrylamide derivative.

### DESCRIPTION OF RELATED ARTS

Sulfonamides compounds exhibit a wide range of biological activities in many aspects, for example, sterilization, weeding, insecticide, anti-cancer and antidiabetic. Therefore, much concern is focused on sulfonamides compounds by researchers of the fields of medicine and pesticide. Unsaturated acrylamide compounds having sulfonamide phenyl substituent can be made into a homopolymer by being homopolymerized with each other or a copolymer by being copolymerized with other monomers having an unsaturated bond, which are widely used in the printing field. The homopolymer with a sulfamine substituent can be used as a primary or additional adhesive for lithographic printing plates, which can improve the performances of chemical resistance, abrasive resistance and printablity of printing plates and so on. The homopolymer is given with a photopolymerization property by inducing unsaturated bond into a side chain thereof. The homopolymer can also be used as an active compound for negative PS plates, CTP thermal plates and processless thermal plates.

CN 102 504 137 A discloses monomers used as co-polymerisation components for thermoplastic nano-micron particles. Compound D5 of this document has a similar structure as the presently claimed compounds, but does not comprise a diazanyl linker between the acrylic moiety and the toluenesulfonamide moiety.

### SUMMARY OF THE PRESENT INVENTION

The main object of the invention is to provide a toluenesulfonamide-substituted acrylamide derivative, which can be used as a comonomer for a copolymer with a sulfamine substituent.

The toluenesulfonamide-substituted acrylamide derivative of the invention has a following structural formula:

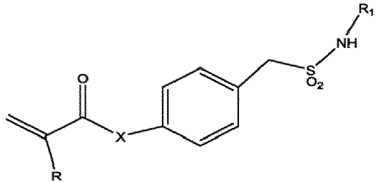

Wherein X represents diazanyl, R represents -H or-CH3, R1 represents C1∼C4 alkyl or aryl. Preferably, R1 represents -CH3.

The toluenesulfonamide-substituted acrylamide derivative of the invention can be made referring to methods well known in the art, for example JP11-044956; USP20100298321; Tetrahedron Letters,50(26),3290-3293,2009; Pharmaceutical Chemistry Journal,46(7),418-428; 2012 and so on.

Wherein the toluenesulfonamide-substituted acrylamide derivative of the invention can be made by the two following methods:

The first method (comparative):

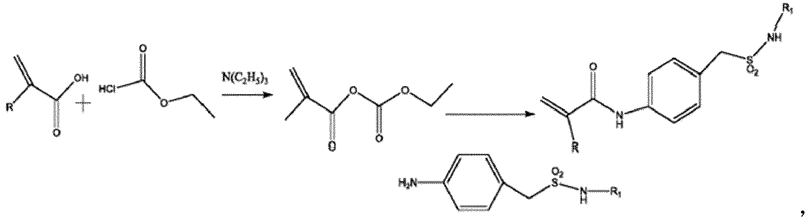

Wherein R represents -H or-CH3, R1 represents C1∼C4 alkyl or aryl. Preferably. The second method:

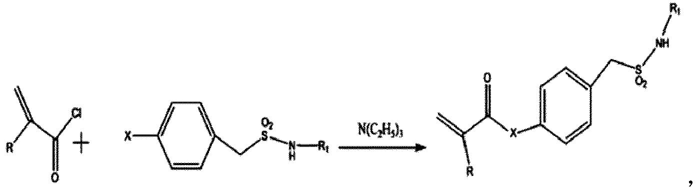

Wherein X represents diazanyl, R represents -H or-CH3, R1 represents C1∼C4 alkyl or aryl.The present invention has the advantages that: (1) the traditional preparation route is adopted, process parameter requirements are not high, the preparation is simple, and only an appropriate reactor is required to be selected; (2) a yield is high, and the refining yield can reach above 80%; and (3) the derivative serves as a monomer for synthesizing a copolymer with a sulfamide substituent, and the synthesized copolymer can serve as a major or additive adhesive for a lithographic plate to improve performances of the lithographic plate, such as chemical resistance, abrasive resistance and development printability of a forme.

### BRIEF DESCRIPTION OF THE DRAWINGS

Figures 1-4 are comparative
Fig.1 is a 1H NMR map of the compound made by the first embodiment.
Fig.2 is an IR map of the compound made by the first embodiment.
Fig.3 is a1H NMR map of the compound made by the second embodiment.
Fig.4 is an IR map of the compound made by the second embodiment.
Fig.5 is a1H NMR map of the compound made by the third embodiment.
Fig.6 is an IR map of the compound made by the third embodiment.

### DETAILED DESCRIPTION OF THE PREFERRED EMBODIMENT

The following combination of the examples and the accompanying drawings further illustrates the present invention. Examples 1 and 2 are comparative.

### Example 1:

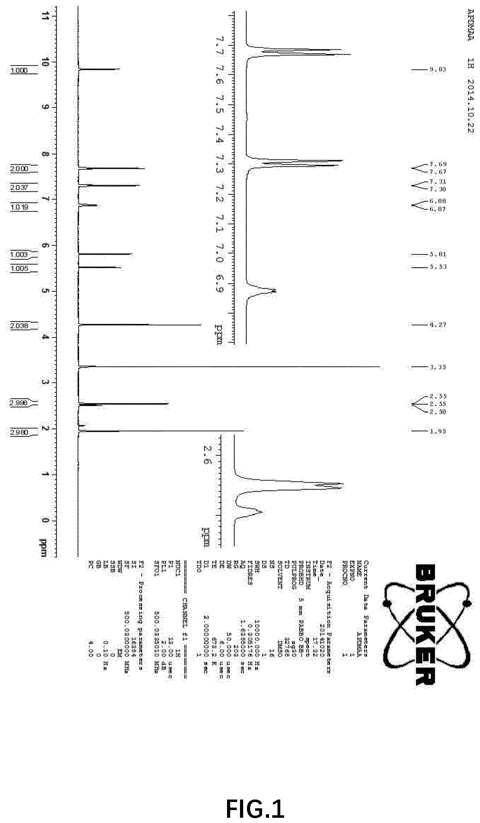

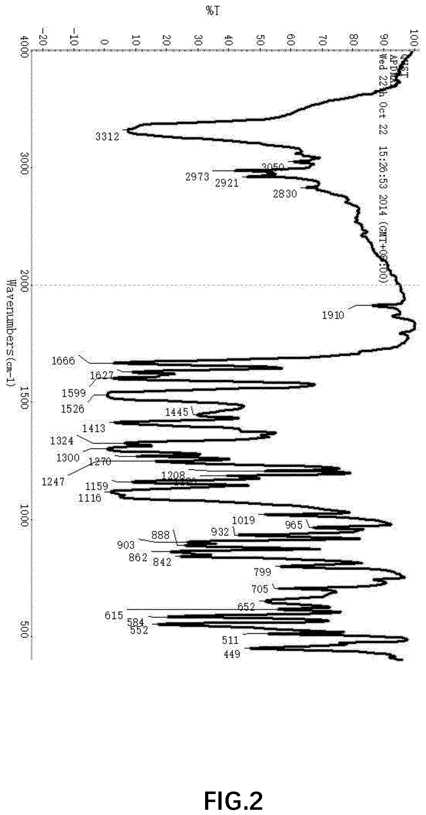

Adding 31.0g (0.36mol) methacrylic acid, 39.1g (0.36mol) ethyl chloroformate and 200ml acetonitrile into a three-mouth flask with a stirrer, a thermometer and a constant pressure funnel, wherein the three-mouth flask has a volume of 500ml, stirring, mixing the mixture. Cooling the mixture by ice-water bathing, and adding dropwise 36.4g(0.36mol) triethylamine. After adding dropwise, removing the ice-water bathing, replacing the constant pressure funnel with a condenser. Stirring for 30min at room temperature. Adding 60.08g(0.30mol) 1-(4-amino phenyl)-N-methyl-methyl-sulfonamide into the reaction mixture, stirring the reaction mixture for 1h at 70 °C. After cooling the reaction mixture, adding the reaction mixture into 1L water and stirring for 30min. And then filtering, beating and washing by utilizing 500ml water, and after drying, 30g N-[4-(N-methyl-amino sulfamoyl)methyl)phenyl] 2-methyl-2-acrylamide is prepared, which is white powder. HPLC(area%): 95.81%, after recrystallizing by acetonitrile, HPLC(area%): 99.09%, mp.189∼190°C.
1H NMR(DMSO)(FIG.1)δ: 1.95(s,3H,-CH₃),2.5∼2.55(dd,3H,-CH₃),4.27(s, 2H,-CH₂-),5.53(s,1H,-CH=),5.81(s,1H,-CH=),6.87∼6.88(m,1H,-SO₂-NH-), 7.3∼ 7.31(d,2H,ArH),7.67∼7.69(d,2H,ArH)9.83(s,1H,-CO-NH).
IR(FIG.2)vcm-1:3312(N-H), 2973(-CH₂-),2921(-CH₂-), 2830(-CH₂-), 1666(Ar-C=O), 1627(N-H), 1599(CO-N), 1526(CO-N), 1413(C-N), 1324 (SO₂-N), 1300(SO₂-N), 1270(CO-NH)1159(SO₂-N), 1116(S=O),862(Ar-p substituted).

### Example 2:

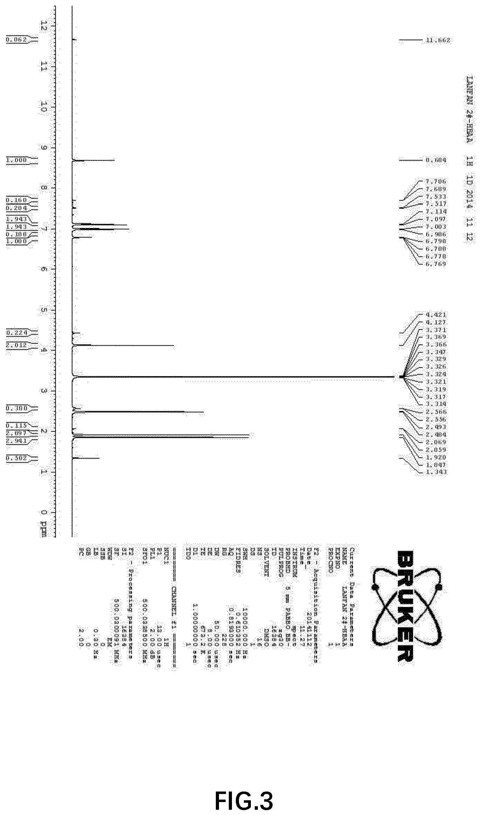

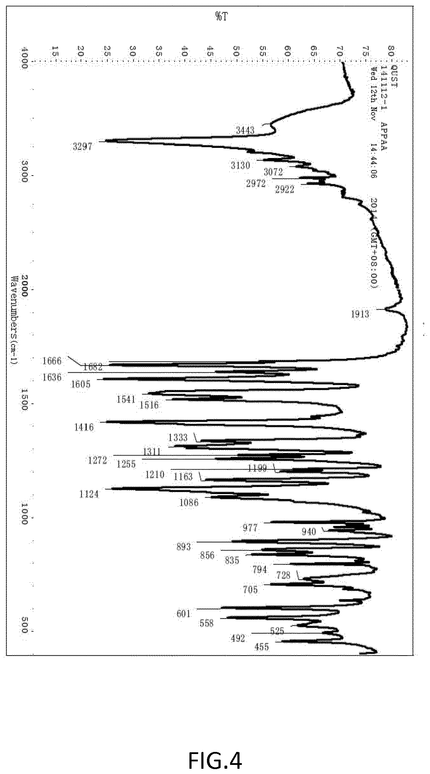

Adding 20.01g(0.1mol) 1-(4-amino-phenyl)-N-methyl-methyl-sulfonamide and 100ml acetone into a three-mouth flask with a stirrer, a thermometer and a constant pressure funnel, wherein the three-mouth flask has a volume of 250ml, stirring and mixing the mixture. Cooling the mixture by ice-salt bathing, and adding dropwise 36.4g(0.36mol) triethylamine. After adding dropwise, removing the ice- salt bathing, replacing the constant pressure funnel with a condenser. Stirring for 30min at room temperature. Cooling the mixture by ice-salt bathing, and adding dropwise the mix solution of 9.05g(0.1mol) acryloyl chloride and 30ml acetone at 0-5°C. After adding dropwise, reacting for 2hours at 0°C, and then removing the ice-water bathing. After removing the ice-water bathing, heating up to the room temperature naturally and stirring for 4hours. And then heating up to 40°C and reacting for 30min. Then heating down to the room temperature, adding the solution of 8.4g sodium bicarbonate and 100g water into the reaction mixture. pH6∼7. Filtering, and water washing twice, and after drying, 19.03g nearly white powder is prepared. HPLC(area%): 97.38%, after recrystallizing by acetonitrile, N-[4-(N-methyl-amino sulfamoyl) methyl)phenyl]-2- acrylamide is prepared and the yield of furfural is 80.5%, which has an appearance of nearly white crystallite. HPLC(area%): 98.7%, mp. 214∼ 216.9°C.
1H NMR(DMSO)(FIG.3)δ: 2.49∼2.54(dd,3H,-CH₃),4.25(s,2H,-CH₂-), 5.74∼5.76(s,1H,-CH=),6.23∼6.27(s,1H,-CH=),6.4∼6.45(s,1H,-CH=), 6.88∼6.89(m,1H,-SO₂-NH-),7.29∼ 7.31(d,2H,ArH),7.64∼7.65(d,2H,ArH) 10.21(s,1H,-CO-NH).
IR FIG.4)vcm-1: 3297(N-H), 2972(-CH₂-),2922(-CH₂-), 1666(Ar-C=O), 1636(N-H), 1605(CO-N), 1541(CO-N), 1416(C-N), 1333(SO₂-N), 1311(SO₂-N), 1272(CO-NH)1163(SO₂-N), 1124(S=O),856(Ar-p substituted).

### Example 3:

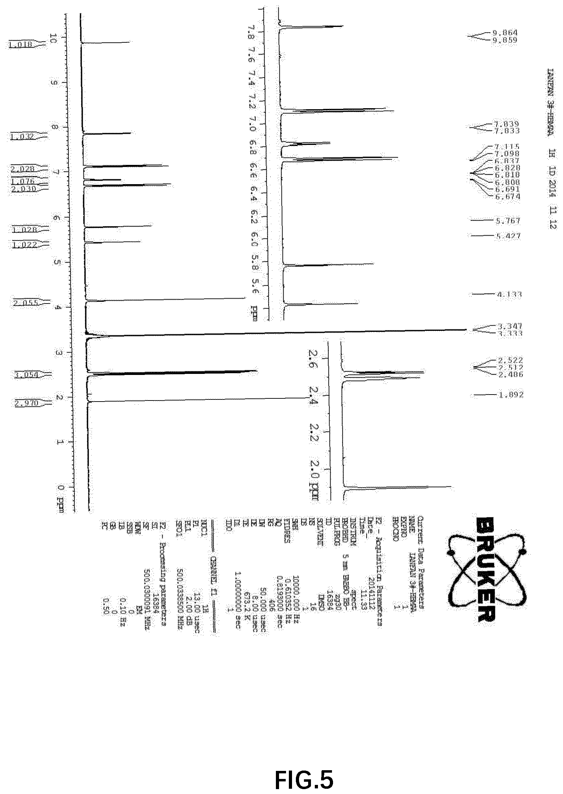

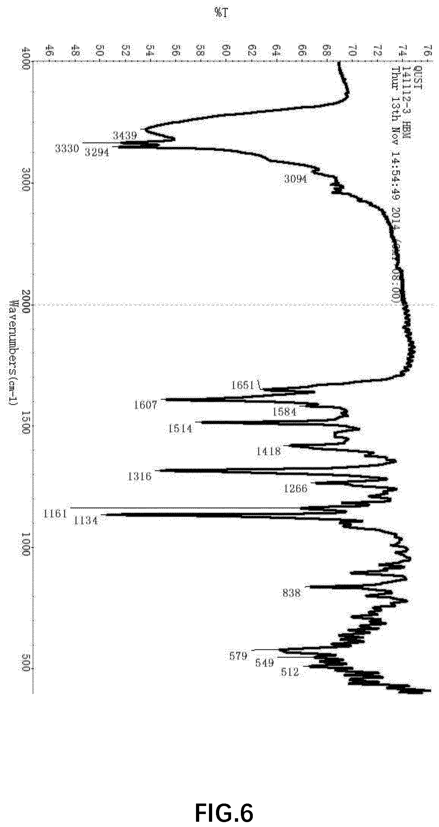

Adding 12.59g(0.1mol) 1-(4-phenyl-hydrazine)-N-methyl-methyl-sulfonamide and 100ml acetone into a three-mouth flask with a stirrer, a thermometer and a constant pressure funnel, wherein the three-mouth flask has a volume of 250ml, stirring and mixing the mixture. Cooling the mixture by ice-salt bathing, and adding dropwise the mixed solution of 5.23g(0.05mol) methacryloyl chloride and 30ml acetone at 0-5°C. After adding dropwise, reacting for 12hours at 0°C. And then, removing the ice-salt bathing, heating up to the room temperature naturally and stirring for 48hours. Then adding the solution of 4.24g sodium bicarbonate and 250g water into the reaction mixture. pF[6∼7. Filtering, and water washing twice, and after drying, 1.91g nearly white powder is prepared. HPLC(area%): 98.94%N-[4-(N-methyl-amino-sulfamoyl)methyl)phenyl]-2-methyl-acrylhydrazine is prepared and mp. 164.3∼166.5°C.
1HNMR(DMSO)(Fig.5)δ: 1.89(s,3H,-CH₃),2.49∼2.52(dd,3H,-CH₃), 4.13(s,2H,-CH₂-),5.43(s,1H,-CH=),5.76(s,1H,-CH=),6.82∼6.84(m,1H,-SO 2-NH-),6.67∼ 6.70(d,2H,ArH),7.1∼7.12(d,2H,ArH), 7.833∼7.839 (d,1H,Ar-NH-),9.859∼9.864(d,1H,-CO-NH).
IR(Fig.6)vcm-1: 3439(N-H), 3297(N-H), 3094(-CH₂-),1651(Ar-C=O), 1607(CON), 1514(CO-N), 1316(SO₂-N), 1161(SO₂-N), 1134(S=O),838(Ar-p substituted).

## Claims

1. A toluenesulfonamide-substituted acrylamide derivative, of the following structural formula: 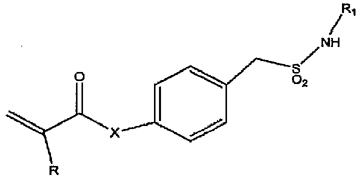 wherein X represents diazanyl, R represents -H or-CH3, R1 represents C1∼C4 alkyl or aryl.

2. The acrylamide derivative, as recited in claim 1, wherein R1 represents -CH3.

## Patentansprüche

1. Toluolsulfonamid-substituiertes Acrylamid-Derivat der folgenden Strukturformel: 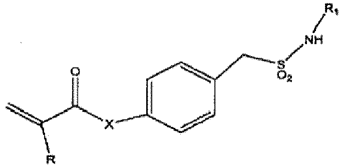 worin X für Diazanyl steht, R für -H oder -CH₃ steht, R₁ für C₁- ∼ C₄-Alkyl oder - Aryl steht.

2. Acrylamid-Derivat nach Anspruch 1, wobei R₁ für -CH₃ steht.

## Revendications

1. Dérivé d'acrylamide substitué avec un toluène-sulfonamide, de la formule structurelle suivante : 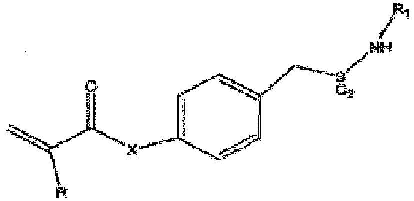 dans lequel X représente un diazanyle, R représente -H ou -CH3, R1 représente un alkyle en Cl à C4 ou un aryle.

2. Dérivé d'acrylamide, selon la revendication 1, dans lequel R1 représente -CH3.
